# FASCICULE DE BREVET EUROPEEN

(11) **EP 2 379 035 B1**
(45) Date de publication et mention de la délivrance du brevet: **25.03.2015**
(21) Numéro de dépôt: 09805761.5
(22) Date de dépôt: 21.12.2009
(51) Int. Cl.: A61F 13/06

(54) **DISPOSITIF DE PROTECTION DU TALON PRÉVENANT L'ESCARRE DU TALON**
FERSENSCHUTZVORRICHTNG ZUR PRÄVENTION VON FERSENSCHORF
HEEL PROTECTION DEVICE FOR PREVENTING A HEEL ESCHAR

(30) Priorité: 22.12.2008 FR 0858923
(43) Date de publication de la demande: 26.10.2011
(73) Titulaire: THUASNE, 92300 Levallois-Perret (FR)
(72) Inventeur: LAFAGE, Danielle, F-18000 Bourges (FR); CONVERT, Reynald, F-42800 Saint Martin la Plaine (FR)
(74) Mandataire: Jacobson, Claude
(86) Numéro de dépôt international: PCT/FR2009/052632
(87) Numéro de publication internationale: WO 2010/072957

(56) Documents cités:
- US-A- 3 990 440
- US-A- 4 292 263
- US-A- 4 705 025

## Description

La présente invention concerne un dispositif de protection du talon du type comprenant une pièce en mousse agencée pour recouvrir le talon lorsque le dispositif est porté et un matériau textile recouvrant la pièce en mousse et maintenant ladite pièce autour du talon lorsque le dispositif est porté, la pièce en mousse étant agencée pour que le dessous et le dessus du pied ne soient pas recouverts par ladite pièce en mousse lorsque le dispositif est porté.

La présente invention concerne plus particulièrement un dispositif permettant de protéger un patient contre l'escarre du talon qui peut survenir lorsque ce patient est immobilisé pendant une longue durée en position allongée.

L'escarre du talon est consécutive à une hypoxie tissulaire, qui est une carence d'apport en oxygène des tissus, aussi bien superficiels que profonds. L'hypoxie tissulaire peut avoir deux origines, à savoir une surpression qui est à l'origine de l'écrasement des capillaires sanguins ou un cisaillement qui peut entraîner des tensions ou des lésions au niveau des capillaires.

Afin de prévenir l'escarre du talon, on connaît des dispositifs protégeant le talon en l'enrobant de mousse afin d'éviter les surpressions sur le talon lorsque celui-ci repose pendant une longue durée sur une surface, telle qu'un lit. Les documents US-A-3 990 440, US-2007/0074427 et US-2007/0073208 décrivent par exemple de tels dispositifs.

Cependant, ces dispositifs sont peu pratiques à mettre en place et nécessite au patient de d'abord positionner correctement son talon dans le dispositif puis de fermer le dispositif autour du pied, ce qui est peu pratique pour une personne immobilisée.

En outre, de tels dispositifs enserrant le pied dans la mousse ne sont pas confortables et encombrants.

L'un des buts de l'invention est de pallier ces inconvénients en proposant un dispositif de protection du talon facile à mettre en place et assurant un confort accru du pied tout en étant peu encombrant.

A cet effet, l'invention concerne un dispositif de protection du talon du type précité, dans lequel le matériau textile est agencé pour recouvrir seul le dessous et le dessus du pied de sorte à fermer le dispositif en formant un manchon fermé de façon à permettre l'enfilage du dispositif à la manière d'une chaussette.

Le dispositif pouvant s'enfiler à la manière d'une chaussette, il est très facile à mettre en place et le talon se positionne correctement à l'intérieur de façon naturelle. En outre, l'utilisateur n'a pas besoin de manipuler de moyens de fermeture pour porter le dispositif. L'intérieur du dispositif n'étant jamais ouvert, celui-ci est relativement protégé des pollutions extérieures, ce qui améliore l'hygiène du dispositif.

Selon d'autres caractéristiques du dispositif :
- la pièce en mousse est agencée pour recouvrir le talon depuis le bas du mollet jusqu'au cuboïde, en couvrant le calcanéum ;
- le matériau textile est élastique ;
- le textile est un tissu ou un tricot élastique ;
- la mousse présente une masse volumique sensiblement comprise entre 38 kg.m⁻³ et 40 kg.m⁻³;
- la mousse présente une résistance à la compression sensiblement égale à 5 kPa pour une déformation de l'ordre de 40% ;
- la pièce de mousse présente une épaisseur sensiblement comprise entre 10 mm et 20 mm ;
- la pièce de mousse comprend un logement de réception du talon à partir duquel s'étendent deux branches destinées à être disposées de part et d'autre du pied sans recouvrir le dessous ou le dessus de celui-ci et une branche destinée à recevoir l'arrière de la jambe sans recouvrir le dessus du pied ;
- la pièce en mousse comprend deux lignes de pliage s'étendant entre la branche de réception de l'arrière de la jambe et les branches s'étendant de part et d'autre du pied, lesdites lignes s'étendant de part et d'autre du talon ; et
- le matériau textile comprend une ligne de pliage reliant les lignes de pliages de la pièce en mousse en passant par le dessus du pied.

D'autres aspects et avantages de l'invention apparaîtront à la lecture de la description qui suit, donnée à titre d'exemple et faite en référence aux dessins annexés, dans lesquels :
- la Fig. 1 est une représentation schématique de côté du dispositif de protection selon l'invention enfilé sur un pied,
- la Fig. 2 est une représentation schématique de dessous du dispositif de protection de la Fig. 1.

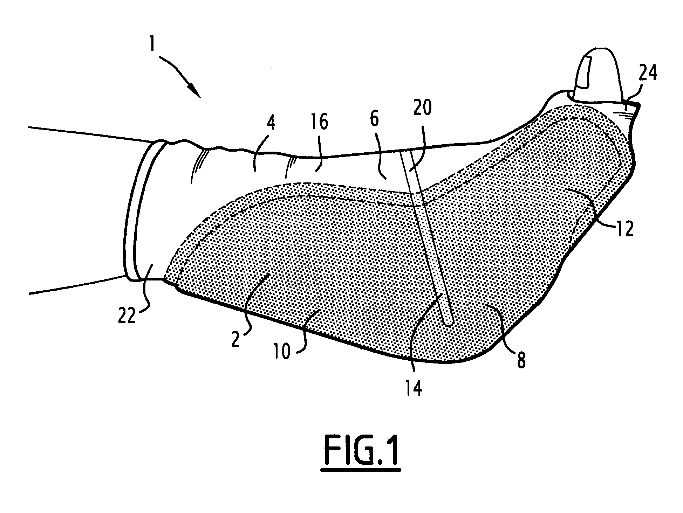

En référence à la Fig. 1, on décrit un dispositif de protection 1 du talon d'un patient, en particulier pour prévenir l'escarre du talon.

Ce dispositif 1 comprend une pièce en mousse 2 et un matériau textile 4 fixés l'un à l'autre de sorte à former un manchon 6 à l'intérieur duquel le patient glisse son pied à la façon d'une chaussette.

Le dispositif 1 est fermé à demeure, c'est-à-dire qu'il ne comprend pas de moyens de fermeture du dispositif 1 autour du pied du patient. On évite ainsi des manipulations fastidieuses du dispositif. En outre, les moyens de fermeture prévus dans les dispositifs de l'art antérieur pendent du dispositif lorsqu'ils ne sont pas utilisés, ce qui est peu pratique pour le rangement du dispositif et peut entraîner des détériorations du dispositif. Le dispositif 1 selon l'invention se manipule d'une pièce et ne nécessite pas d'opération de fermeture ou de serrage.

La pièce en mousse 2 est agencée pour recouvrir le talon depuis le bas du mollet jusqu'au cuboïde, en couvrant le calcanéum. A cet effet, la pièce en mousse 2 comprend un logement coudé 8 de réception du talon. Le logement 8 comprend une branche 10 s'étendant derrière la jambe du patient et sur les côtés de celle-ci sans recouvrir le dessus de la jambe et du pied. A l'opposé de la branche 10, le logement 8 comprend en outre deux branches 12 s'étendant de part et d'autre du pied sans recouvrir le dessous ou le dessus du pied. Afin de conférer la forme coudée au logement 8, la pièce en mousse 2 comprend deux lignes de pliage 14 entre la branche 10 et les branches 12 de la pièce 2. Les lignes 14 s'étendent de part et d'autre du talon et autorisent une déformation la pièce en mousse 2 afin de permettre l'enfilage du dispositif 1. La pièce en mousse 2 présente une certaine élasticité de sorte que le logement 8 reprend sa forme initiale coudée une fois que le dispositif a été enfilé.

La pièce en mousse 2 présente une densité et une résilience adaptée de sorte à diminuer les surpressions qui s'exercent localement au niveau du talon. A cet effet, la mousse présente par exemple une masse volumique sensiblement comprise entre 38 kg.m⁻³ et 40 kg.m⁻³, une résistance à la compression sensiblement égale à 5 kPa pour une déformation de l'ordre de 40% et une épaisseur sensiblement comprise entre 10 mm et 20 mm. Une telle pièce de mousse permet de protéger efficacement le talon contre l'escarre.

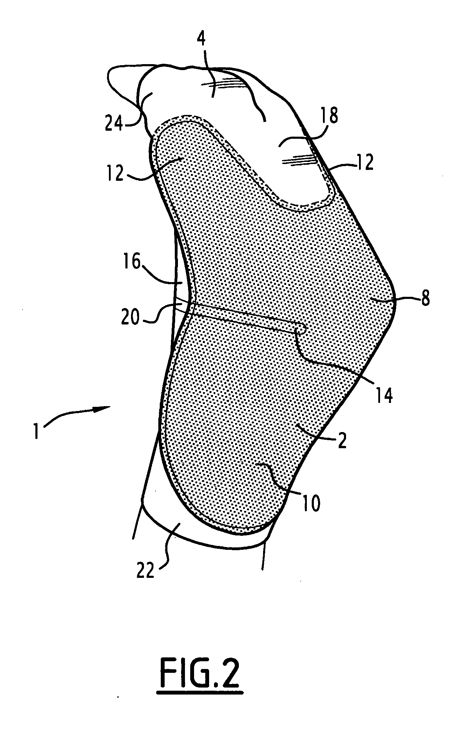

Le matériau textile 4 recouvre la pièce en mousse 2 et relie les bords de la branche 10 ainsi que les branches 12 entre elles de sorte à former le manchon 6. A cet effet, le matériau textile comprend une partie supérieure 16 reliant les bords de la branche 10 et les branches 12 en passant par le dessus du pied et une partie inférieure 18 (visible sur la Fig. 2), reliant les branches 12 en passant par le dessous du pied. Ainsi, le matériau textile 4 recouvre le dessus et le dessous du pied où la mousse est absente et permet de former un dispositif 1 pouvant s'enfiler à la manière d'une chaussette. La partie supérieure 16 du matériau textile 4 comprend une ligne de pliage 20 reliant les deux lignes de pliages 14 de la pièce en mousse 2 en passant par le dessus du pied, ce qui autorise la déformation du dispositif 1 pour permettre de l'enfiler. Le matériau textile 4 comprend en outre deux parties extrêmes 22 et 24 de section sensiblement circulaire s'étendant de part et d'autre de la pièce en mousse 2, la partie extrême 22 s'étendant au-dessus de la branche 10 et faisant le tour du mollet du patient et la partie extrême 24 s'étendant derrière les branches 12 et faisant le tour du bout du pied du patient, en laissant les orteils à l'air libre.

Le matériau textile 4 est légèrement élastique et permet de serrer le dispositif 1 autour du talon du patient sans nécessiter de moyen de serrage, à la manière d'une chaussette.

Le matériau textile 4 est un tissu ou un tricot élastique en matière artificielle ou naturelle agencée pour limiter le cisaillement des capillaires sanguins et ainsi éviter les troubles trophiques.

Le fait de prévoir que seul le textile 4 recouvre le dessus et le dessous du pied permet de faciliter l'enfilage du dispositif 1 qui est ainsi rendu plus souple en limitant la présence de mousse aux endroits où elle est vraiment nécessaire.

Le dispositif 1 est prévu en plusieurs tailles adaptées aux différentes dimensions de pieds. Ces tailles sont adaptées pour que le dispositif 1 n'exerce qu'une faible pression sur le dessus du pied, une trop grande pression étant susceptible d'augmenter les risques d'escarre.

Le dispositif 1 décrit ci-dessus permet d'augmenter la surface de contact du talon avec le support sur lequel il repose, telle qu'un matelas ou une table. Par exemple, cette surface passe de 15 cm² à 45 cm², voire jusqu'à 60 cm2. La pression exercée au niveau du talon est ainsi grandement diminuée et passe, à titre d'exemple, de 150, ou 200, mmHg à moins de 100 mmHg, ce qui réduit fortement les risques d'escarre du talon.

Comme précédemment détaillé, le dispositif 1 peut s'enfiler de façon simple sans nécessiter de manipulation fastidieuse de moyens de fermeture ou de moyen de serrage. En outre, le dispositif 1 est peu encombrant. L'hygiène offerte par le dispositif est également améliorée du fait que la face interne du dispositif n'est pas accessible, ce qui évite à des éléments étrangers de se loger dans le logement 8 de réception du talon.

Un tel dispositif, dans une construction similaire à celle destinée à l'escarre du talon, i.e. avec une pièce de mousse et une partie textile, peut-être déclinée en manchon, cagoule ou shorty, par exemple, avec comme application la prévention des escarres au niveau du coude, de la tête ou du sacrum et du trochanter.

## Revendications

1. Dispositif de protection (1) du talon du type comprenant une pièce en mousse (2) agencée pour recouvrir le talon lorsque le dispositif est porté et un matériau textile (4) recouvrant la pièce en mousse (2) et maintenant ladite pièce (2) autour du talon lorsque le dispositif est porté, la pièce en mousse (2) étant agencée pour que le dessous et le dessus du pied ne soient pas recouverts par ladite pièce en mousse (2) lorsque le dispositif est porté, le matériau textile (4) étant agencé pour recouvrir seul le dessous et le dessus du pied de sorte à fermer le dispositif en formant un manchon (6) fermé de façon à permettre l'enfilage du dispositif à la manière d'une chaussette, **caractérisé en ce que** la pièce de mousse (2) comprend un logement (8) de réception du talon à partir duquel s'étendent deux branches (12) destinées à être disposées de part et d'autre du pied sans recouvrir le dessous ou le dessus de celui-ci et une branche (10) destinée à recevoir l'arrière de la jambe sans recouvrir le dessus du pied.

2. Dispositif de protection du talon selon la revendication 1, **caractérisé en ce que** la pièce en mousse (2) est agencée pour recouvrir le talon depuis le bas du mollet jusqu'au cuboïde, en couvrant le calcanéum.

3. Dispositif de protection du talon selon la revendication 1 ou 2, **caractérisé en ce que** le matériau textile (4) est élastique.

4. Dispositif de protection du talon selon l'une quelconque des revendications 1 à 3, **caractérisé en ce que** le textile (4) est un tissu ou un tricot élastique.

5. Dispositif de protection du talon selon l'une quelconque des revendications 1 à 4, **caractérisé en ce que** la mousse (2) présente une masse volumique sensiblement comprise entre 38 kg.m⁻³ et 40 kg.m⁻³.

6. Dispositif de protection du talon selon l'une quelconque des revendications 1 à 5, **caractérisé en ce que** la mousse (2) présente une résistance à la compression sensiblement égale à 5 kPa pour une déformation de l'ordre de 40%.

7. Dispositif de protection du talon selon l'une quelconque des revendications 1 à 6, **caractérisé en ce que** la pièce de mousse (2) présente une épaisseur sensiblement comprise entre 10 mm et 20 mm.

8. Dispositif de protection du talon selon l'une quelconque des revendications 1 à 7, **caractérisé en ce que** la pièce en mousse (2) comprend deux lignes de pliage (14) s'étendant entre la branche (10) de réception de l'arrière de la jambe et les branches (12) s'étendant de part et d'autre du pied, lesdites lignes (14) s'étendant de part et d'autre du talon.

9. Dispositif de protection du talon selon la revendication 8, **caractérisé en ce que** le matériau textile (4) comprend une ligne de pliage (16) reliant les lignes de pliages (14) de la pièce en mousse (2) en passant par le dessus du pied.

## Patentansprüche

1. Vorrichtung zum Schutz (1) der Ferse des Typs aufweisend ein Schaumstück (2), das eingerichtet ist, um die Ferse zu überdecken, wenn die Vorrichtung getragen wird, und ein Textilmaterial (4), das das Schaumstück (2) überdeckt und das das besagte Stück (2) um die Ferse hält, wenn die Vorrichtung getragen wird, wobei das Schaumstück (2) eingerichtet ist, damit die Unterseite und die Oberseite des Fußes nicht von dem besagten Schaumstück (2) überdeckt werden, wenn die Vorrichtung getragen wird, wobei das Textilmaterial (4) eingerichtet ist, um nur die Unterseite und die Oberseite des Fußes zu überdecken in der Weise, dass die Vorrichtung geschlossen wird unter Bildung einer geschlossenen Muffe (6), so dass das Anziehen der Vorrichtung auf Weise eines Strumpfes ermöglicht ist, **dadurch gekennzeichnet, dass** das Schaumstück (2) eine Unterbringung (8) zur Aufnahme der Ferse aufweist, von welcher ausgehend sich zwei Arme (12), die dazu bestimmt sind, beiderseits des Fußes angeordnet zu sein, ohne dessen Unterseite oder dessen Oberseite zu überdecken, und ein Arm (10) erstrecken, der dazu bestimmt ist, die Hinterseite des Beins aufzunehmen, ohne die Oberseite des Fußes zu überdecken.

2. Vorrichtung zum Schutz der Ferse gemäß Anspruch 1, **dadurch gekennzeichnet, dass** das Schaumstück (2) eingerichtet ist, um die Ferse vom Unterteil der Wade aus bis zum Würfelbein hin zu überdecken unter Überdecken des Fersenbeins.

3. Vorrichtung zum Schutz der Ferse gemäß Anspruch 1 oder 2, **dadurch gekennzeichnet, dass** das Textilmaterial elastisch ist.

4. Vorrichtung zum Schutz der Ferse gemäß irgendeinem der Ansprüche 1 bis 3, **dadurch gekennzeichnet, dass** das Textil (4) ein elastisches Gewebe oder Gewirk ist.

5. Vorrichtung zum Schutz der Ferse gemäß irgendeinem der Ansprüche 1 bis 4, **dadurch gekennzeichnet, dass** der Schaum (2) eine Dichte von im Wesentlichen zwischen 38 kg/m³ und 40 kg/m³ hat.

6. Vorrichtung zum Schutz der Ferse gemäß irgendeinem der Ansprüche 1 bis 5, **dadurch gekennzeichnet, dass** der Schaum (2) eine Druckfestigkeit von im Wesentlichen gleich 5kPa bei einer Deformation in der Größenordnung von 40% hat.

7. Vorrichtung zum Schutz der Ferse gemäß einem der Ansprüche 1 bis 6, **dadurch gekennzeichnet, dass** das Schaumstück (2) eine Dicke von im Wesentlichen zwischen 10mm und 20mm hat.

8. Vorrichtung zum Schutz der Ferse gemäß irgendeinem der Ansprüche 1 bis 7, **dadurch gekennzeichnet, dass** das Schaumstück (2) zwei Faltlinien (14) hat, die sich zwischen dem Arm (10) zur Aufnahme der Hinterseite des Beins und den Armen (12) erstrecken, die sich beiderseits des Fußes erstrecken, wobei die besagten Linien (14) sich beiderseits der Ferse erstrecken.

9. Vorrichtung zum Schutz der Ferse gemäß Anspruch 8, **dadurch gekennzeichnet, dass** das Textilmaterial (4) eine Faltlinie (16) hat, die die Faltlinien (14) des Schaumstücks (2) verbinden unter Passieren der Oberseite des Fußes.

## Claims

1. Device (1) for protecting the heel, of the type comprising a foam piece (2) designed to cover the heel when the device is worn and a textile material (4) covering the foam piece (2) and holding said piece (2) around the heel when the device is worn, the foam piece (2) being designed so that the bottom and the top of the foot are not covered by said foam piece (2) when the device is worn, the textile material (4) being designed to cover only the bottom and top of the foot so as to close the device forming a closed sleeve (6) to enable the device to be pulled on in the manner of a sock, **characterised in that** the foam piece (2) comprises a compartment (8) for receiving the heel, from which extend two branches (12) intended to be disposed on either side of the foot without covering the bottom or the top thereof and a branch (10) intended to receive the back of the leg without covering the top of the foot.

2. Heel protection device as claimed in claim 1, **characterised in that** the foam piece (2) is designed to cover the heel from the lower part of the calf to the cuboid bone, covering the calcaneus.

3. Heel protection device as claimed in claim 1 or 2, **characterised in that** the textile material (4) is elastic.

4. Heel protection device as claimed in any one of claims 1 to 3, **characterised in that** the textile (4) is an elastic fabric or knitted fabric.

5. Heel protection device as claimed in any one of claims 1 to 4, **characterised in that** the foam (2) has a density substantially between 38 kg·m⁻³ and 40 kg·m⁻³.

6. Heel protection device as claimed in any one of claims 1 to 5, **characterised in that** the foam (2) has a resistance to compression substantially equal to 5 kPa for a deformation in the order of 40%.

7. Heel protection device as claimed in any one of claims 1 to 6, **characterised in that** the foam piece (2) has a thickness substantially between 10 mm and 20 mm.

8. Heel protection device as claimed in any one of claims 1 to 7, **characterised in that** the foam piece (2) has two fold lines (14) extending between the branch (10) receiving the back of the leg and the branches (12) extending on either side of the foot, said lines (14) extending on either side of the heel.

9. Heel protection device as claimed in claim 8, **characterised in that** the textile material (4) has a fold line (16) passing underneath the foot linking the fold lines (14) of the foam piece (2).
